# EUROPEAN PATENT APPLICATION

(11) **EP 1 108 714 A1**
(43) Date of publication of application: **20.06.2001**
(21) Application number: 00310749.7
(22) Date of filing: 04.12.2000
(51) Int. Cl.: C07D 277/56, A01N 43/78

(54) **Microbicidal salts of 5-carboxanilido-haloalkylthiazoles**

(30) Priority: 16.12.1999 US 171235 P
(71) Applicant: ROHM AND HAAS COMPANY, Philadelphia, Pennsylvania 19106-2399 (US)
(72) Inventor: Michelotti, Enrique Luis, Fort Washington, Pennsylvania 19034 (US); Bryman, Lois Merle, North Wales, Pennsylvania 19454 (US)
(74) Representative: Buckley, Guy Julian

(57) **Abstract**

Compounds of the formula: 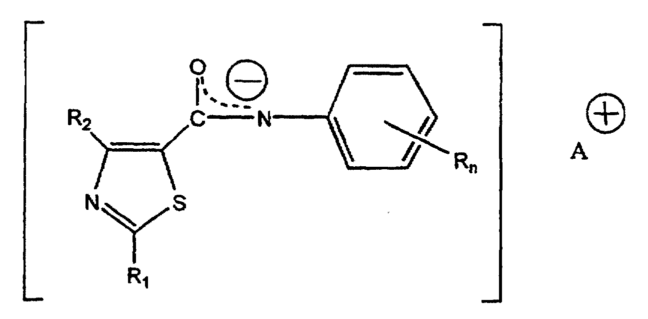 wherein
A is a strong base cation;
R₁ and R₂ are independently (C₁-C₅)alkyl or (C₁-C₂)haloalkyl, provided that at least one of R₁ and R₂ is (C₁-C₂)haloalkyl;
each R is independently halo, halo(C₁-C₅)alkyl, halo(C₁-C₅)alkoxy, nitro, cyano, pentahalosulfur, halomethylthio, haloethylthio, (C₁-C₂)alkylsulfinyl, halo(C₁-C₂)alkylsulfinyl, (C₁-C₂)alkylsulfonyl, or halo(C₁-C₂)alkylsulfonyl; and
n is from one to five,
are useful as fungicides, microbicides, marine antifoulants, and termiticides, compositions containing those compounds, and methods for their use.

## Description

This invention relates to salts of 5-carboxanilido-haloalkylthiazoles which are useful as fungicides, microbicides, marine antifoulants, and termiticides, compositions containing those compounds, and methods for their use.

U. S. Patent No. 5,045,554 discloses a class of substituted 5-carboxanilidothiazoles useful for control of plant fungus disease such as, for example *Basidiomycetes* such as *Rhizoctonia, Sclerotium,* and *Corticium,* as well as *Alternaria* and *Spirothica,* when applied to the growing plant, preferably as a foliar spray. However, there remains a need for such compounds with improved biological activity and/or physical/chemical properties for formulation purposes. We have discovered that certain salts of such compounds provide surprisingly improved biological activity over those exemplified in U. S. Patent No. 5,045,554. This provides a significant advantage to the grower because less fungicide needs to be applied for control of fungal diseases on the grower's crops. Furthermore, such salts are also useful as microbicides, marine antifoulants, and termiticides. In addition, because such salts are often water soluble, commercial formulations are generally easier to prepare and at a lower cost than those of the known 5-carboxanilidothiazoles.

The present invention provides compounds of Formula I: 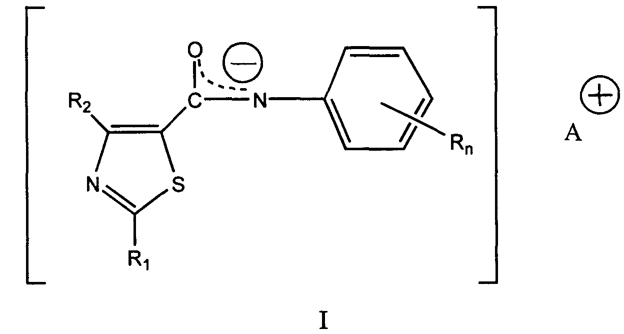 wherein A is a base cation, preferably a cation of a strong base, more preferably a sodium, potassium, lithium, diazabicycloundecene, or diazabicyclononane cation, most preferably a sodium, potassium, or lithium cation; R₁ is (C₁-C₂)haloalkyl or (C₁-C₅)alkyl, preferably (C₁-C₂)alkyl, most preferably methyl; R2 is (C₁-C₅)alkyl or (C₁-C₂)haloalkyl, preferably halomethyl, more preferably perhalomethyl, most preferably trifluoromethyl, provided that at least one of R₁ and R₂ is a (C₁-C₂)haloalkyl; each R is independently halo (preferably chloro, bromo, or iodo), halo(C₁-C₅)alkyl (preferably halo(C₁-C₂)alkyl, more preferably perhalomethyl, most preferably trifluoromethyl), halo(C₁-C₅)alkoxy (preferably halo(C₁-C₂)alkoxy, more preferably perhalomethoxy, most preferably trifluoromethoxy), nitro, cyano, pentahalosulfur (preferably pentafluorosulfur), halomethylthio, haloethylthio, (C₁-C₂)alkylsulfinyl, halo(C₁-C₂)alkylsulfinyl, (C₁-C₂)alkylsulfonyl, or halo(C₁-C₂)alkylsulfonyl; and n is from one to five (preferably two to four, more preferably three to four, most preferably three). Preferably each R is independently halo, haloalkyl, or haloalkoxy. Preferably, the R groups are located in the ortho or para positions. Preferably the para substituent, when present, also has lipophilic character.

The base cation results from reaction by a base with the compound of formula I in which the base abstracts at least one proton from the compound of formula I. Preferably the base has a pKₐ of 10 or more, more preferably the base has a pKₐ of 12 or more. Most preferably the base is sodium hydroxide, potassium hydroxide, or lithium hydroxide, or a mixture thereof.

The term "carboxanilido" means C₆H₅-NH-CO-. The term "alkyl" means straight or branched chain (C₁-C₅)alkyl, unless otherwise specified. The term "lipophilic" means having an affinity for organic solvents rather than aqueous solvents. The term "active ingredient" means a compound of Formula I. For purposes of this invention, all percentages are percent by weight, unless otherwise specified, all percentage ranges are inclusive, all ratios are by weight, and all ratio ranges are inclusive.

A second embodiment of this invention is for compositions of the compounds of Formula I which are prepared by admixing the compound with one or more adjuvants including diluents, extenders, carriers, surfactants, and conditioning agents to provide compositions in the form of particulate solids, solutions, dispersions, or emulsions. Such compositions include, for example, aqueous solutions, wettable powders, granulars, dusts, emulsifiable concentrates, and flowables.

The compounds of Formula I can be applied to various loci such as the soil, growing agricultural crops, ornamental crops, turf, structures, and industrial sites. When used as fungicides, compounds of Formula 1 can be used to inhibit the growth of fungi by introducing a fungicidally effective amount of the compound onto, into, or at a locus where fungi grow including, but not limited to soil, growing agricultural crops, ornamental crops, and turf.

When used as microbicides, compounds of Formula I can be used to inhibit the growth of microorganisms by introducing a microbicidally effective amount of one or more of the agents onto, into, or at a locus subject to microbial attack. Suitable loci include, but are not limited to: cooling towers; air washers; boilers; mineral slurries; wastewater treatment; ornamental fountains; reverse osmosis filters; ultrafilters; ballast water; evaporative condensers; heat exchangers; pulp and paper processing fluids; plastics; emulsions and dispersions; paints; latexes; coatings, such as varnishes; construction products, such as mastics, caulks, and sealants; construction adhesives, such as ceramic adhesives, carpet backing adhesives, and laminating adhesives; industrial or consumer adhesives; photographic chemicals; printing fluids; household products, such as bathroom disinfectants or sanitizers; cosmetics and toiletries; shampoos; soaps; detergents; industrial disinfectants or sanitizers, such as cold sterilants, hard surface disinfectants; floor polishes; laundry rinse water; metalworking fluids; conveyor lubricants; hydraulic fluids; leather and leather products; textiles; textile products; wood and wood products, such as plywood, chipboard, flakeboard, laminated beams, oriented strandboard, hardboard, and particleboard; petroleum processing fluids; fuel; oil field fluids, such as injection water, fracture fluids, and drilling muds; agriculture adjuvant preservation; surfactant preservation; medical devices; diagnostic reagent preservation; food preservation, such as plastic or paper food wrap; pools; and spas. Preferred loci are wood and wood products, cooling towers; air washers; boilers; mineral slurries; wastewater treatment; ornamental fountains; reverse osmosis filtration; ultrafiltration; ballast water; evaporative condensers; heat exchangers; pulp and paper processing fluids; plastics; emulsions and dispersions; paints; latexes; and coatings. Depending upon the locus, the compounds of the present invention may be either directly incorporated into the locus, applied directly to the locus, or incorporated into a coating which is then applied to the locus.

When used as marine antifouling agents, compounds of the present invention can be used to control or inhibit the growth of marine organisms by application of a marine antifouling effective amount of their compositions onto or into a marine structure. Depending upon the particular marine structure to be protected, the compositions of the present invention can be directly incorporated into the marine structure, applied directly to the marine structure, or incorporated into a coating which is then applied to the marine structure. Suitable marine structures include, but are not limited to: boats, ships, oil platforms, piers, pilings, docks, elastomeric rubbers, and fish nets and line. The compositions of the present invention are typically directly incorporated into structures such as elastomeric rubber or fish net fibers during manufacture. Direct application of the compositions of the invention is typically made to structures such as fish nets or wood pilings. The compositions of the invention can also be incorporated into a marine coating, such as a marine paint or varnish.

Certain of the compounds of Formula I, specifically those in which both R₁ and R₂ are trifluoromethyl groups, may also be used to combat termites in soil, crops, grassland, forestry, and in other cellulose-based materials. For such purposes these compounds can be used in the technical or pure form as prepared or as formulated compositions.

Solid compositions include, for example: wettable powders typically containing, for example, from 10 to 90%, preferably from 50 to 90% active ingredient, from 2 to 10% dispersing agents, optionally up to 10% stabilizers and/or other additives such as penetrants, stickers, and surfactants, and a solid inert carrier such as clay, silica, or natural or synthetic carrier; dusts which are usually formulated as a concentrate having a composition similar to a wettable powder but without dispersant and usually containing from 0.5 to 10% active ingredient; granules containing, for example, from 0.01 to 80% active ingredient and optionally up to 10% additives such as stabilizers, surfactants, slow release modifiers, and binding agents which are prepared by, for example, agglomeration or impregnation techniques and have a size greater than wettable powders and up to 1-2 millimeters; and baits containing, for example, from 0.01 to 25% active ingredient prepared by combining the active ingredient with a cellulose-based material and other additives. Liquid compositions include, for example, aqueous or solvent based solutions, emulsifiable concentrates, emulsions, suspension concentrates, and flowables which typically contain from 0.01 to 99.9 percent by weight of the active ingredient, an acceptable carrier, and one or more adjuvants. More typically such liquid compositions will contain from 1.0 to 85% of the active ingredient.

It is usually desirable, particularly in the case of sprayable formulations, to include one or more adjuvants, such as wetting agents, spreading agents, dispersing agents, stickers, adhesives, emulsifying agents and the like in accordance with agricultural practices. Such adjuvants commonly used in the art can be found in *McCutcheon's Emulsifiers and Detergents, McCutcheon's Emulsifiers and Detergents/Functional Materials,* and *McCutcheon's Functional Materials* all published annually by McCutcheon Division of MC Publishing Company (New Jersey) and *Farm Chemicals Handbook* published annually by Meister Publishing Company (Ohio).

The effective amount of compound needed to provide the required level of control of fungal disease, microbes, marine fouling organisms, or termites will vary depending upon one or more of the following factors: the growing or environmental conditions; the susceptibility of plants to fungal diseases which are to be controlled; the environment in which the microbe or marine fouling organism to be controlled is found;, the composition used; phytotoxic effects of the composition on the plant; and the specific compound or composition employed. Under typical conditions, fungal diseases are controlled at application rates of 30 to 500 grams/hectare, microbes are controlled at application rates which provide from 0.5 to 2500 ppm of the compound at the locus to be protected, marine fouling organisms are controlled at rates of 0.1 to 30 percent, based on the weight of the structure to be protected or based on the weight of compound incorporated into a coating to be applied to a marine structure, and the effective dosage of the compound for control of termites is of the order of 0.0001 to 2.0% based on the total weight of a composition applied to the locus of the termites. One skilled in the art can readily determine the optimum amount of compound or composition required based upon the above noted factors.

The compounds and compositions of this invention can be diluted or applied as is to plant foliage and/or soil as aqueous sprays by methods commonly employed, such as conventional high-volume hydraulic sprays, low-volume sprays, air-blast, and aerial sprays. The dilution and rate of application will depend upon the type of equipment employed, the method and frequency of application desired, the application rate, and the fungal disease, microbe, or termites to be controlled. The compositions can be mixed with fertilizers or fertilizing materials before their application. The compositions can be utilized as the sole pesticidal agent or they can be employed in conjunction with other pesticidal agents such as, for example, microbicides, marine antifoulants, fungicides, herbicides, insecticides, and acaricides.

Application onto or into wood or timber may be accomplished using conventional techniques such as immersion of the timber into a liquid composition, painting by spraying or brushing, dipping, or injecting or impregnating the composition into the timber. For such applications, the concentration of the compound of Formula I in the composition should be sufficient to provide an effective amount of the compound in or on the timber.

In the present invention, the compounds of Formula I are generally synthesized by reacting an appropriately substituted thiazole having a 5-carbonylchloride substituent with an appropriately substituted aniline in suitable solvents at an elevated temperature according to the procedures described in U. S. Patent No. 5,045,554, hereby incorporated by reference, and then converting the product of such a reaction into a salt by reaction with a strong base. Suitable solvents for the first reaction include, for example, xylene, tetrahydrofuran, toluene, chlorobenzene, collidine, and 2,6-di-t-butyl-4-methyl-pyridine.

The 2,4-bis-trifluoromethylthiazole-5-carboxanilides are prepared using procedures analogous to the following:

### Preparation of 2,4-bis-trifluoromethylthiazole-5-carboxvlic acid chloride

### Step 1 - Preparation of trifluorothioacetamide:

To a 1L 4-neck round bottom flask (RBF), equipped with a mechanical stirrer, nitrogen inlet, addition funnel and thermometer, was charged trifluoroacetamide (56.0 grams (g), 1.0 equiv. 0.495 mole) and 100 g of Lawesson's reagent followed by 500 milliliters (mL) of tetrahydrofuran. The reaction mixture was heated to boiling for 2 hours. The solvent was carefully removed by rotary evaporation to yield 86 g of crude product. This material was distilled by kugelrohr distillation under high vacuum (<1 mm Hg) to afford 54 g of light yellow liquid trifluorothioacetamide (84% yield).

### Step 2 - Preparation of ethyl chlorotrifluoroacetoacetate:

To a 500 mL 3-neck RBF equipped with a magnetic stirrer, nitrogen inlet, thermometer and gas bubbler was charged 200 g of ethyl trifluoroacetoacetate. Using an acetone/ice bath the reaction vessel was cooled to 0-10 °C and at this temperature chlorine gas was added to the reaction vessel via a gas bubbler at sufficient rate to maintain the reaction from 5 to 15 °C. Chlorine gas was added until a yellow color persisted in the reaction mixture. The reaction solution was allowed to warm to room temperature and then heated to 30C while gas was evolved. When the gas evolution stopped, the resulting mixture provided 226 g of product (95% yield).

### Step 3 - Preparation of ethyl 2,4-bis-trifluoromethyl-5-thiazole carboxylate:

To a 3L 4-neck RBF equipped with a mechanical stirrer, reflux condenser, thermometer and addition funnel was charged 358 g of ethyl chlorotrifluoroacetoacetate (1.64 moles), 2,2,2-trifluorothioacetamide and 1000 mL of acetonitrile. To this mixture was added 331.9 g of triethylamine (2.0 eq, 3.28 moles) dropwise over 2.5 hours. During the addition the temperature was maintained at 30-38 °C and upon completion of the addition the reaction was heated to reflux for 2 hours and stirred overnight at room temperature. The reaction mixture was filtered and the resulting filtrate was concentrated by rotary evaporation to provide an oily solid which was dissolved in 1500 mL of ethyl acetate. This was washed with 2 x 500 mL of water, 1 x 500 mL of brine and concentrated by rotary evaporation to yield 356.6 g of ethyl 2,4-bis-trifluoromethyl-5-thiazole carboxylate which was purified by distillation.

### Step 4 - Preparation of 2,4-bis-trifluoromethylthiazole-5-carboxylic acid:

To a 1L 4-neck RBF was charged ethyl 2,4-bis-trifluoromethyl-5-thiazole carboxylate (23.8 g, 1.0 equiv., 81.2 mmole) in 100 mL THF and 50 mL water. The reaction mixture was cooled to 20 °C and 10% NaOH solution (32.5 g, 1.0 equiv., 81.2 mmole) was added. The ice-bath was removed after 5 minutes and the mixture was stirred for 4 hours. After reaction was complete, as determined by thin layer chromatography, 100 mL ether and 100 mL water were added. The aqueous phase was separated and acidified with conc. HCl, extracted with ether, and the ether was removed by rotary evaporation to give a solid which was washed with water and vacuum filtered. The solid was dried in a vacuum oven to give 16.5 g (76.7% yield) product as a brown solid, mp = 98-101 °C.

### Step 5 - Preparation of 2,4-bis-trifluoromethylthiazole-5-carboxylic acid chloride:

To a 500 mL 1-neck RBF under N2 was added 2,4-bis-trifluoromethyl-thiazole-5-carboxylic acid (31.5 g, 1.0 equiv., 0.119 moles) in 25 mL chloroform and 1 mL dimethylformamide (DMF). To this solution was added thionyl chloride (28.3 g, 2.0 equiv., 0.24 moles). The reaction was then heated at reflux for 6 hours. The reaction was cooled to room temperature and concentrated by rotary evaporation at 30 °C to remove solvent. Chloroform was added, 3 x 25 mL portions, concentrating by rotary evaporation each time, to give 29.8g (88.4% yield) product as a brown oil.

### Aniline Coupling Reactions

### Preparation of 135,660 N-(2,4,6-trichlorophenyl)-2,4-bis-trifluoromethylthiazole-5-carboxanilide:

To a 250 mL 1-neck RBF under nitrogen was added 2,4-bis-trifluoromethyl-thiazole-5-carboxylic acid chloride(25.8 g, 1.0 equiv., 91.0 mmoles) in 30 mL toluene and then 2,4,6-trichloroaniline (17.9 g, 1.0 equiv., 91.0 mmoles). The mixture was heated at reflux for 6 hours with monitoring by gas-liquid chromatography (GLC). Upon completion the reaction was cooled to room temperature. A dark colored solid formed upon cooling after residual toluene was evaporated. The dark colored solid was washed with methylene chloride, vacuum filtered, and further washed with hexanes to give 33.2 g (82.2% yield) product as an off-white solid, mp = 180-182 °C.

### Preparation of Thifluzamide Sodium Salt

Aqueous sodium hydroxide (0.32 mL of 50 percent solution) was added to a mixture of 2.55 grams (4.8 mmol.) of thifluzamide (5-(2,6-dibromo-4-trifluoromethoxycarboxanilido)-2-methyl-4-trifluoromethyl-1,3-thiazole) and 10 mL of water. The resulting solution was stirred for 1 hour at room temperature and then gravity filtered. The water was removed from the filtrate by vacuum rotary evaporation leaving 2.5 grams of the sodium salt as a light brown solid (¹H-NMR (D₂O) d 2.73 (3H, s), 7.65 (2H, s). The solid was dried in a vacuum oven at low heat before using.

### Example 1 - Diffusion

The purpose of this test was to examine differences in the diffusion rate in agar between thifluzamide and its sodium salt using the fungus, *Sclerotium rolfsii.*

Potato dextrose agar (PDA) plates were prepared in a two-step procedure as follows: Four batches of PDA were prepared and adjusted to pH 5.0 with dilute HCl and to pH 7.0 and 9.0 with dilute NaOH, followed by autoclaving and pouring. Petri plates were filled approximately 30% full of molten agar and allowed to cool. After the agar solidified, one plastic microscope coverslip was placed in the center of the plate. Several hours later, the petri plates were filled to 70% of their capacity, trapping the coverslip in the center of agar plate. One day later, six sclerotia of the fungus *Sclerotium rolfsii* were placed 1 cm from the edge of the petri plate in an equidistant pattern from the center. After three days, the sclerotia started to produce mycelium on the agar surface. A cork borer was used to remove a 0.8-cm diameter agar plug from the center of the plate. The coverslip barrier prevented the cork borer from penetrating past the midway point of the agar depth.

Thifluzamide sodium salt solutions and thifluzamide formulated as a suspension concentrates in sterile distilled water were prepared at concentrations of 10,000 and 1,000 ppm. Approximately 0.7 ml of each solution and suspention was pipetted into the center well of each PDA plate. Two replications were used for each treatment.

After 1 and 2 weeks from the time the sclerotia were placed on the PDA surface, measurements of the diameter of the zone of inhibition were recorded. Three measurements were made, each one representing the distance between the edge of mycelial growth from sclerotia opposite each other across the center well. The area in square millimeters (mm²) of the zone of inhibition was also determined.

One week after sclerotia were placed on the plates, the average diameter of the zone of inhibition around the center well was approximately 33% larger in plates filled with the sodium salt, as compared to the suspension concentrate formulation, for both the 10,000 and 1,000 ppm rates. The 1,000 ppm plates containing the sodium salt produced a similar size zone of inhibition as did the 10,000 ppm plates containing the suspension concentrate thifluzamide. Thus, the sodium salt appears to be approximately 10 times more active in this agar diffusion assay than the suspension concentrate thifluzamide.

After two weeks at the 10,000 ppm rate, the zones of inhibition produced by the sodium salt were approximately 113, 99, and 36% larger at pH 9,7, and 5, respectively, than the zones produced by the suspension concentrate thifluzamide.

The agar diffusion test showed that the sodium salt of thifluzamide possesses different properties than the thifluzamide itself. In an agar diffusion test, the sodium salt produced a larger zone of inhibition by preventing colonies of *S*. *rolfsii* from overgrowing the area. Based on the similar size of the zones of inhibition, the sodium salt of thifluzamide at 1,000 ppm diffused into an area equal to the zone produced by 10,000 ppm thifluzamide. Hence the sodium salt appeared to be approximately 10 times more active.

### Example 2 - Biological Evaluation

In the following examples the biological activity of four different compounds, thifluzamide (Cmpd. 1) and its sodium, potassium, and lithium salts (Cmpds. 2, 3, and 4), was evaluated. The chemicals for testing were all technical material, >95% active ingredient. Each compound was diluted with a 2:1:1 (v/v) mixture of water, acetone, and methanol, to achieve the appropriate concentrations, beginning at 300 g/ha.

Test plants were grown under greenhouse conditions in a peat moss and vermiculite soil-less mix, except rice plants that were grown in 50% mix and 50% sterilized soil (v/v). All plants were planted in 2¼ x 2¼ inch plastic pots.

The solution was sprayed onto the plants and allowed to dry for two hours. Then the plants were inoculated with fungal spores. Each test utilized control plants which were sprayed with the solvent mixture and inoculated. For these protective tests, the plants inoculated with the two powdery mildews were inoculated the same day as spraying. All other inoculations were performed one day after spraying the plants with the compound. After spraying, the plants were stored at room temperature under low light until inoculation.

The remainder of the technique of each of the tests is given below along with the results for various compounds described herein. The results are percent disease control are compared to the untreated check, wherein one hundred was rated as complete disease control and zero as no disease control. The application of the fungal spores to the treated test plants to induce the following plant diseases was as follows:

### 1. Cucumber Anthracnose (CA)

The fungal pathogen *Colletotrichum lagenarium* was cultured on potato dextrose agar (PDA) in the dark at 22°C for a period of 8 to 14 days. Spores of *C. lagenarium* were removed from the PDA plates by flooding the plate surface with distilled water, amended with 0.5% v/w of yeast extract. The upper surface of the fungal colony was scraped with a blunt instrument until most of the spores were released into the aqueous environment. The spore suspension was filtered though cheesecloth, and the spore count was adjusted to 3.0 x 10⁶ spores/mL.

The chemically-treated cucumber plants were 15-days old, cultivar Bush Champion. The upper leaf surface of the plants was sprayed with the spore suspension just prior to runoff, using a hand-held pump spray bottle. The plants were placed in a fluorescent-lighted mist chamber (12 hr light, 12 hr dark) for 48 hours. After that infection period, the plants were placed in a growth chamber for 3 more days at 25°C and 90% humidity. The treated plants were then evaluated for disease control.

### 2. Cucumber Powdery Mildew (CPM)

A culture of powdery mildew *Sphaerotheca fulginea* was maintained on large cucumber plants, cultivar Bush Champion, in the greenhouse. Inoculum was prepared by placing five to ten heavily mildewed leaves in a glass jar with 500 mL of water containing five drops of Tween-80 surfactant. After shaking the liquid and leaves to release the spores, the suspension was filtered through cheesecloth, and the spore count was adjusted to 100,000 spores/mL.

The upper leaf surface of the plants was sprayed with the spore suspension just prior to runoff, using a hand-held pump spray bottle. The plants were maintained in the greenhouse for infection and disease development. Seven days after inoculation, the plants were evaluated for percent disease control.

### 3. Rice Blast (RB)

Cultures of *Pyricularia oryzae* were maintained on potato dextrose agar (PDA) for two to three weeks. Spores of *P. oryzae* were removed from the PDA plates by flooding the plate surface with distilled water, amended with Tween-80 at 1 drop per 100 mL distilled water. The upper surface of the fungal colony was scraped with a blunt instrument until most of the spores were released into the aqueous environment. After filtering the spore suspension through two layers of cheese cloth, the spore count was adjusted to 5 x 10⁵ spores/mL.

The spore suspension was sprayed onto 12-day old rice plants, cultivar M-201, using a DeVilbiss atomizer. Each pot of rice plants, containing 20 to 30 plants, received approximately 1 mL of inoculum. The inoculated plants were placed in a dark humidity cabinet at 20°C for 36 hours to allow for infection. After the infection period, the plants were placed in the greenhouse. After 6 more days, the plants were evaluated for percent disease control.

### 4. Wheat Leaf Rust (WLR)

The fungal pathogen *Puccinia recondita f.* sp. *tritici* was maintained by inoculating 7-day old wheat plants, cultivar Fielder. Approximately two-weeks later, spores were collected from the leaves by scraping the plants over aluminum foil to collect the spores. The spores were cleaned by sieving through a 250-micron size screen and stored dry. The dried spores were used within one month.

A spore suspension was prepared from dry uredia spores by adding 20 mg (9.5 million spores) per mL of Soltrol oil. The suspension was dispensed into gelatin capsules (0.7 mL capacity) which attach to the oil atomizers. One capsule was used for twenty pots of wheat, each pot containing 20 to 30 plants of the cultivar Fielder that were 7-days old. After waiting for at least 15 minutes for the oil to evaporate from the wheat leaves, the plants were placed in a dark mist chamber at 20°C for 24 hours. The plants were then placed in the greenhouse and evaluated after an additional 12 days for percent disease control.

### 5. Wheat Powdery Mildew (WPM)

The fungal pathogen *Erysiphe graminis* f. sp. *tritici* was cultured on wheat seedlings, cultivar Fielder, by inoculating 7-day old plants. After 8 days in a controlled temperature room at 18°C, the wheat powdery mildew spores were shaken directly from the culture plants onto 7-day old wheat seedlings which had been previously treated with experimental compounds. The inoculated seedlings were kept in a controlled temperature room at 18°C and 80% humidity to allow for disease development. The percent disease control was evaluated seven days after inoculation.

### 6. Grape Downy Mildew (GDM)

The fungal pathogen *Plasmopara viticola* was cultured on small grape plants derived from plant tissue culture from the cultivar Delaware. Infected leaves producing spores were collected and frozen, until the spores were needed. Treated plants were sprayed with a spore suspension in water, containing 200,000 spores/mL. The plants were placed in an unlit humidity cabinet at 20°C for 24 hours. After that infection period, the plants were transferred to a growth chamber at 18°C and 90% humidity to allow for disease development. The percent disease control was evaluated seven days after inoculation.

Fungicidal activity against the above discussed phytopathogenic fungi is set forth in the following table expressed as percent disease control.

| **Cmpd.** | **Rate*** | **CA** | **RB** | **TLB** | **WLR** | **WPM** | **CPM** |
|---|---|---|---|---|---|---|---|
| 1 | 300 | 0 | 0 | 0 | 100 | 85 | 100 |
| | 75 | 0 | 0 | 0 | 99 | 80 | 99 |
| | 19 | 0 | 0 | 0 | 95 | 50 | 75 |
| 2 | 300 | 0 | 0 | 0 | 100 | 85 | 100 |
| | 75 | 0 | 0 | 0 | 99 | 75 | 99 |
| | 19 | 0 | 0 | 0 | 95 | 50 | 75 |
| 3 | 300 | 0 | 0 | 0 | 100 | 85 | 100 |
| | 75 | 0 | 0 | 0 | 99 | 85 | 95 |
| | 19 | 0 | 0 | 0 | 95 | 50 | 80 |
| 4 | 300 | 0 | 0 | 0 | 100 | 85 | 100 |
| | 75 | 0 | 0 | 0 | 99 | 85 | 99 |
| | 19 | 0 | 0 | 0 | 90 | 0 | 80 |

| | | | | | | | |
|---|---|---|---|---|---|---|---|
| *Application rate is expressed in parts per million (ppm) | | | | | | | |

These data indicate that at low rates, the salt form of the compound is surprisingly as efficacious as the compound itself for control of several test organisms. Coupled with the fact that the salts are water soluble whereas the compounds themselves are not, the salts provide advantages because they can be formulated as aqueous formulations. In addition, the salts may have handling advantages in the manufacturing process because of their crystallinity and high melting points.

## Claims

1. A compound of the formula: 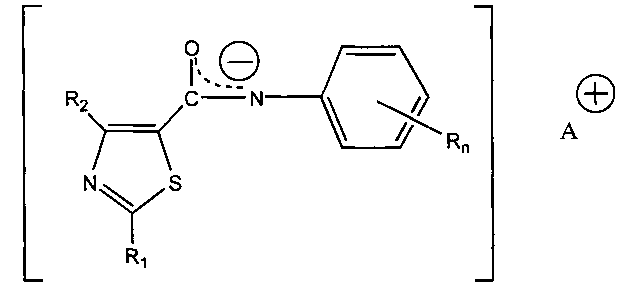 wherein:
A is a strong base cation;
R₁ and R₂ are independently (C₁-C₅)alkyl or (C₁-C₂)haloalkyl, provided that at least one of R₁ and R₂ is (C₁-C₂)haloalkyl;
each R is independently halo, halo(C₁-C₅)alkyl, halo(C₁-C₅)alkoxy, nitro, cyano, pentahalosulfur, halomethylthio, haloethylthio, (C₁-C2)alkylsulfinyl, halo(C₁-C₂)alkylsulfinyl, (C₁-C₂)alkylsulfonyl, or halo(C₁-C₂)alkylsulfonyl; and
n is from one to five.

2. The compound of claim 1, wherein: R₁ is (C₁-C₂)alkyl; R₂ is halomethyl; each R is independently halo, halo(C₁-C₅)alkyl, or halo(C₁-C₅)alkoxy; and n is 2, 3, or 4.

3. The compound of claim 1, wherein: R₁ is methyl; R₂ is trifluoromethyl; each R is independently bromo or trifluoromethoxy; and n is 3.

4. The compound of claim 1, wherein: R₁ is methyl; R₂ is trifluoromethyl; n is 3, and the R groups are 2,5-dibromo-4-trifluoromethoxy.

5. The compound of claim 1, wherein A is sodium or potassium.

6. A fungicidal composition comprising:
a) a compound of the formula: 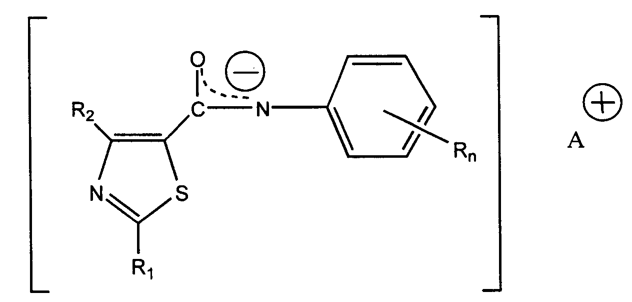 wherein:
A is a strong base cation;
R₁ and R₂ are independently (C₁-C₅)alkyl or (C₁-C₂)haloalkyl, provided that at least one of R₁ and R₂ is (C₁-C₂)haloalkyl;
each R is independently halo, halo(C₁-C₅)alkyl, halo(C₁-C₅)alkoxy, nitro, cyano, pentahalosulfur, halomethylthio, haloethylthio, (C₁-C₂)alkylsulfinyl, halo(C₁-C₂)alkylsulfinyl, (C₁-C₂)alkylsulfonyl, or halo(C₁-C₂)alkylsulfonyl; and
n is from one to five; and
b) one or more acceptable carriers.

7. A method for controlling a fungus disease comprising applying to a locus of the fungus a fungicidally effective amount of one or more compositions of claim 6.

8. A method for controlling a microorganism comprising introducing a microbicidally effective amount of one or more compositions of claim 6 onto, into, or at a locus subject to microbial attack.

9. A method for controlling a marine organism comprising introducing a marine antifoulant effective amount of one or more compositions of claim 6 onto or into a marine structure.

10. A method for controlling termites comprising applying to a locus of the termites a termiticidally effective amount of one or more compositions of claim 6.
